# EUROPEAN PATENT APPLICATION

(11) **EP 2 158 858 A1**
(43) Date of publication of application: **03.03.2010**
(21) Application number: 09175834.2
(22) Date of filing: 16.09.2004
(51) Int. Cl.: A61B 17/22

(54) **Embolectomy device**

(30) Priority: 17.09.2003 US 664134
(62) Divisional of application: 04784163.0
(71) Applicant: Boston Scientific Limited, St. Michael (BB)
(72) Inventor: The designation of the inventor has not yet been filed
(74) Representative: Vossius & Partner

(57) **Abstract**

An embodiment is a catheter comprising a first elongate shaft (302) having a proximal end, a distal end and a first lumen therethrough, a wire (314) having a proximal end and a distal end at least partially disposed in the first elongate shaft, the distal end extending distally from the first elongate shaft, and a motion control apparatus connected to the proximal end of the wire, further comprising a device to the distal end of the wire for changing the shape of an embolus, wherein the device is configured to change the shape of the embolus to unclog a distal catheter lumen.

## Description

### Field of the Invention

The present invention relates generally to the field of intravascular devices. More specifically, the present invention pertains to embolectomy devices for aspirating foreign bodies within a body lumen.

### Background of the Invention

There are a number of situations in the practice of medicine where it is desirable to remove an embolus from a patient's vasculature. If an embolus is not removed it may travel to the neural vasculature, for example, and cause severe trauma. Many prior art embolectomy devices require a retrieval portion to be placed downstream or distal the embolus. This is not always practical or desirable. Other prior art embolectomy devices may require the use of a significant vacuum to remove the embolectomy. This may cause the collapse of a portion of the vasculature and result in trauma.

### Summary of the Invention

In one embodiment of an embolectomy device, a first catheter having an expandable tip may be disposed inside of a second catheter which constrains the tip. The proximal end of either the first or second catheters may be fluidly attached to a vacuum source. The tip may be expanded by moving the first catheter distally relative the second catheter. An embolus may then be urged into the tip by operating the vacuum source.

In another embodiment of an embolectomy device, a first catheter having an expandable tip may be disposed inside of a second catheter which constrains the tip. A clot pulling device may be disposed within the second catheter. The tip may be expanded by moving the first catheter distally relative the second catheter. The clot pulling device may be operated to urge an embolus into the expanded tip.

In another embodiment, a clot unclogging or fragmenting device may be disposed in a catheter, which may be fluidly connected to a vacuum source. The unclogging or fragmenting device may be connected to a motion control apparatus by a wire disposed in a lumen of the catheter. The unclogging or fragmenting device may be operated to open the tip of a catheter blocked by the clot burden or to fragment an embolus, which may then be drawn into a catheter lumen by operation of the vacuum source. The catheter may have a lumen connected to an irrigation source.

The above summary of some embodiments is not intended to describe each disclosed embodiment or every implementation of the present invention. The figures and detailed description which follow more particularly exemplify these embodiments.

The following items are preferred embodiments of the invention. {Please insert pages 2a-2c}

### Brief Description of the Drawings

The invention may be more completely understood in consideration of the following detailed description of various embodiments of the invention in connection with the accompanying drawings in which:
Figure 1a depicts an embolectomy device 300 disposed in a body lumen.
Figure 1b depicts an embolectomy device 400 disposed in a body lumen.
Figure 1c depicts an embolectomy device 500 disposed in a body lumen.
Figure 2a depicts a retrieval catheter 204 of embolectomy device 200.
Figure 2b depicts a guide catheter 206 of embolectomy device 200.
Figure 2c depicts embolectomy device 200.
Figure 2d depicts embolectomy device 200.
Figure 3 depicts an embolectomy device 100 disposed in a vascular lumen.

1. A catheter for changing the shape of an embolus comprising:
   a first elongate shaft having a proximal end, a distal end and a lumen therethrough;
   a second elongate shaft at least partially disposed in the lumen of the first elongate shaft, the second shaft having a proximal end, a distal end and a lumen therethrough;
   a tip disposed on the distal end of the second shaft having a cavity fluidly connected to the lumen of the second shaft and a distal opening, the tip movable between a first state and a second state wherein the distal opening has a greater cross-sectional area in the second state than in the first state; and
   a vibratable wire for changing the shape of an embolus at least partially disposed within the lumen of the second elongate shaft.
2. The catheter of item 1, wherein the wire is configured to unclogged the lumen of the second elongate shaft.
3. The catheter of item 1, wherein the wire is configured to fragment the embolus.
4. The catheter of item 1, wherein the cavity has a greater volume in the second state than in the first state.
5. The catheter of item 1, wherein the distal opening is proximal the distal end of the first elongate shaft in the first state and wherein the distal opening is distal the distal end of the first elongate shaft in the second state.
6. The catheter of item 1, wherein the second elongate shaft comprises a shape memory polyurethane.
7. The catheter of item 1, wherein the second elongate shaft comprises a nitinol coiled sheet catheter.
8. The catheter of item 1, wherein the second elongate member comprises an expandable braid.
9. The catheter of item 1, further comprising a vacuum source fluidly connected to the distal end of the first shaft.
10. The catheter of item 1, further comprising a vacuum source fluidly connected to the distal end of the second shaft.
11. The catheter of item 1, further comprising a clot pulling device disposed within the lumen of the second elongate shaft.
12. The catheter of item 11, further comprising a third elongate shaft having a lumen at least partially disposable in the lumen of the second elongate shaft, the clot pulling device at least partially disposable in the lumen of the catheter.
13. The catheter of item 1, wherein in the second state the distal opening has a cross-sectional area that is larger than the cross-sectional area of the lumen of the first elongate shaft at the distal end.
14. A catheter comprising:
   a first elongate shaft having a proximal end, a distal end and a first lumen therethrough;
   a wire having a proximal end and a distal end at least partially disposed in the first elongate shaft, the distal end extending distally from the first elongate shaft; and
   a motion control apparatus connected to the proximal end of the wire.
15. The catheter of item 14, wherein the motion control apparatus can impart a vibrating motion to the wire.
16. The catheter of item 15, wherein the vibrating motion has a frequency less than about 20 kHz.
17. The catheter of item 16, wherein the vibrating motion has a frequency of between about 1 Hz and about 150 Hz.
18. The catheter of item 15, wherein the vibrating motion is axial.
19. The catheter of item 14, further comprising a device attached to the distal end of the wire for changing the shape of an embolus.
20. The catheter of item 19, wherein the device is configured to change the shape of the embolus to unclog a distal catheter lumen.
21. The catheter of item 19, wherein the device is configured to fragment an embolus.
22. The catheter of item 19, wherein the device is an arcuate wire.
23. The catheter of item 19, wherein the device is a wire having a zigzag shape.
24. The catheter of item 19,wherein the device is a loop.
25. The catheter of item 19, wherein the device has a working range of about 20 mm proximally and about 100 mm distally.
26. The catheter of claim 25 wherein the device has a working range of about 2 mm proximally and about 15 mm distally.
27. The catheter of item 20, further comprising a vacuum source fluidly connected to the distal end of the first elongate shaft.
28. The catheter of item 27, further comprising a second elongate lumen disposed in the first elongate shaft, the wire at least partially disposed in the second lumen.
29. The catheter of item 14, wherein the distal end of the first elongate shaft proximate the first lumen is angled.

### Detailed Description

The following detailed description should be read with reference to the drawings, in which like elements in different drawings are numbered identically. The drawings which are not necessarily to scale, depict selected embodiments and are not intended to limit the scope of the invention.

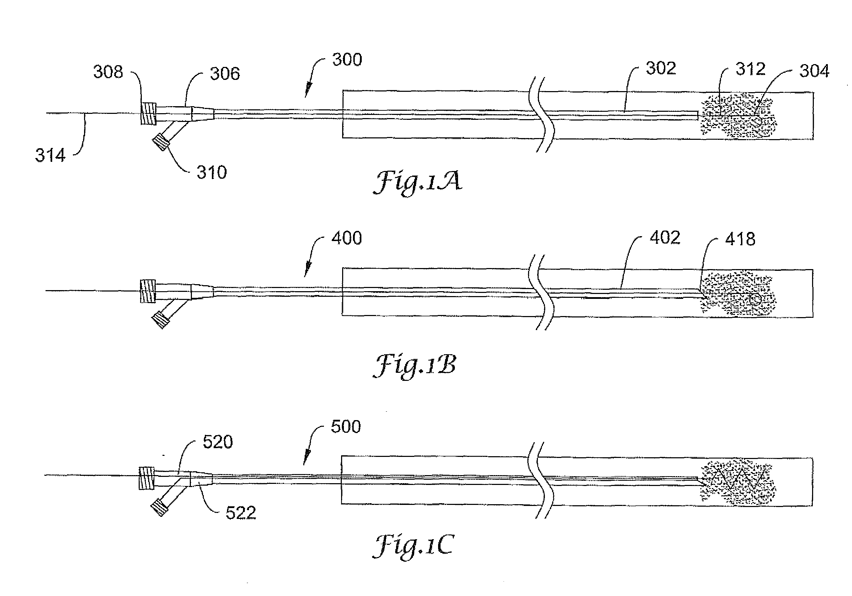

Figure 1a depicts an embolectomy device 300 disposed in a body lumen. Device 300 includes catheter 302 and distal device 304. Distal device 304 may be used to unclog the aspiration lumen or to fragment an embolus for aspiration. Catheter 302 may have a manifold 306 attached proximally including a first port 308 and a second port 310. Distal device 304 has a proximal end 312 attached to an elongate member 314 disposed in a lumen of catheter 302. Distal device 304 may have an arcuate shape, or may be formed into a loop, coil, paddle, whisk, zigzag, helical or other shape suitable for fragmenting an embolus. The proximal end of elongate member 314 may be free or may be attached to a motion control apparatus able to impart motion along the axis of elongate member 314. The motion control apparatus may impart longitudinal or radial motion or vibration to the distal end of elongate member 314. Catheter 302 may also be fluidly attached to a vacuum source.

The motion control apparatus may impart a motion to distal device 304 at between 1 Hz and 150 Hz. Of course, motion at higher or lower frequencies than this are envisioned. As an example, it may be advantageous to move distal device 304 at selective intervals lower than 1 Hz only when a lumen is clogged. In addition, it may be preferable to impart a motion at up to 20 kHz. The motion control apparatus may have any advantageous range of motion. One example range of motion is 17 mm. This may be done by configuring the motion control apparatus to move distal device 2 mm proximally and 15 mm distal from a starting position. Another example range of motion is 120 mm, with the motion control apparatus configured to move distal device 304 20 mm proximally and 100 mm distally.

Figure 1b depicts an embolectomy device 400. Device 400 is similar to device 300 and includes a catheter 402 having an angled distal end 418.

Figure 1c depicts an embolectomy device 500. Device 500 is similar to device 300 and includes a first lumen 520 and a second lumen 522. Elongate member 314 is disposed in first lumen 520 and the vacuum source is fluidly connected to second lumen 522. In use, embolectomy device 500 may be positioned proximate an embolus and the vacuum source may be operated. Distal device 304 may be operated, either by hand or through a motion control apparatus to unclog an aspiration or other lumen or to fragment an embolus. Distal device 304 may thereby fragment the embolus and the embolus or one or more fragments thereof is drawn into second lumen 522. Distal device 304 may alter the shape of an embolus and unclog a lumen or fragment the embolus through vibrations or pulses at the distal end of elongate member 314. In an alternative use, fluid may be irrigated through first lumen 520 or through an additional lumen. Distal device 304 may alternatively or additionally be used to unclog an embolus from a lumen by removing the embolus burden and thereby creating an open channel for more effective aspiration

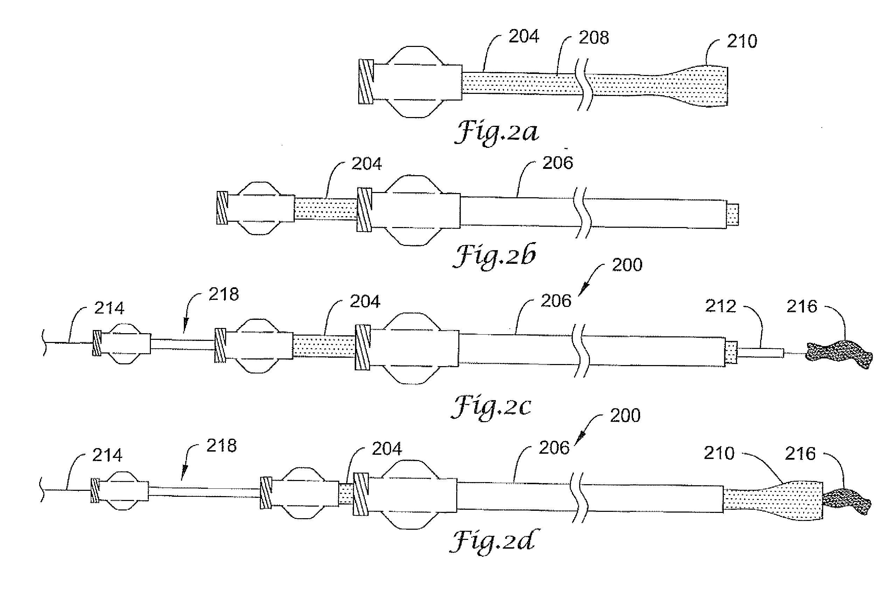

Figure 2c depicts embolectomy device 200, which includes retrieval sheath 204 and guide catheter 206. As depicted in Figure 2a, retrieval sheath 204 may include an expandable elongate shaft or elongate shaft 208 and expandable tip portion 210. Expandable tip portion may be formed from a shape memory polyurethane, a nitinol coiled sheet catheter, an expanding nitinol mesh or braid or other suitable material. A coiled sheet catheter may be fashioned from a flat ribbon of nitinol or other suitable material by coiling the ribbon so that proximal coils overlap and thereby constrain distal coils. When unconstrained, expandable tip portion 210 has an expanded profile and an expanded distal lumen. As shown in Figure 2b, expandable tip portion 210 may also be constrained to fit within guide catheter 206. Embolectomy device 200 may include a clot pulling device 212, comprising an elongate member 214 and wire mesh 216 or other suitable embolus capturing device. Clot pulling device 212 may include and be disposed in a microcatheter 218. In one contemplated method, retrieval sheath 204 may be disposed in guide catheter 206 so that the distal ends are approximately even and are located proximate an embolus. Clot pulling device 212 then may be inserted through sheath 204 to capture or retain the embolus. Catheter 206 then may be moved proximally so that tip portion 210 is distally disposed of guide catheter 206 and expands as shown in Figure 2d. Alternatively, retrieval sheath 204 may be moved distally relative guide catheter 206 to expand tip portion 210. Clot pulling device 212 may then be moved to position the embolus into the expanded tip portion 210. Retrieval sheath 204, and clot pulling device 212 may then be removed proximally from guide catheter 206. If desired, the embolic material may be removed from retrieval sheath 204 and clot pulling device 212 and these devices may be reintroduced into guide catheter 206. Of course other methods are contemplated. For instance, retrieval sheath 204 may be urged distally to cause tip portion 210 to expand and then clot pulling device 212 is inserted distally through retrieval sheath 204.

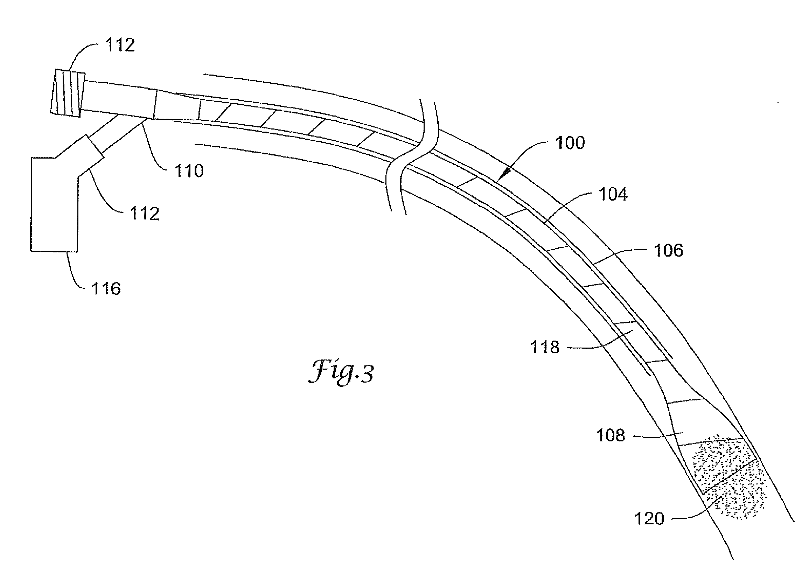

Figure 3 depicts an embolectomy device 100 in use in a vascular lumen 102. Device 100 includes a retrieval catheter 104 and a sheath catheter 106. Retrieval catheter 104 includes lumen 118 and may have an unconstrained state where its profile has a greater cross sectional area than the profile of sheath catheter 106 or may have a tip portion 108 having an unconstrained profile having a greater cross sectional area than the profile of sheath catheter 106. Retrieval catheter 104 also has a constrained state where it may be disposed within sheath catheter 106. Retrieval catheter 104 may be fluidly coupled to a vacuum source 116 and may include a proximally positioned manifold 110 for this purpose. Manifold may include one or more axially or radially located ports 112. Retrieval catheter includes an expandable material such as a shape memory polyurethane, nitinol coiled sheet catheter, or other suitable material. In use, retrieval catheter 104 is disposed in the lumen of sheath catheter 106 and is positioned proximate an embolus 120. Retrieval catheter 104 may be extended distally or sheath catheter 106 may be retracted proximally until a desired distal portion of retrieval catheter 104, which may include tip portion 108, is in an expanded state. Vacuum source 116 may be operated to urge embolus 120 into lumen 118. Alternatively, retrieval catheter 104 having an expanded distal portion may be positioned to capture embolus 120 in lumen 118 and vacuum source 116 may be operated to secure the embolus. Once the embolus is capture, it may be removed. This may be accomplished by retracting retrieval catheter 104 proximally into sheath catheter 106 or by extending sheath catheter 106 distally. Tip portion 108 may be fully or partially disposed within sheath catheter 106. Embolectomy device 100 may then be removed from vascular lumpen 102. Alternatively, retrieval catheter 104 alone may be removed distally from sheath catheter 106. In another alternative, vacuum source 116 may be operated to remove embolus 120 distally from retrieval catheter 104. In another alternative an irrigation catheter may be used to provide fluid.

Numerous advantages of the invention covered by this document have been set forth in the foregoing description. It will be understood, however, that this disclosure is, in many respects, only illustrative. Changes may be made in details, particularly in matters of shape, size, and arrangement of parts or order of steps without exceeding the scope of the invention. The invention's scope is, of course, defined in the language in which the appended claims are expressed.

## Claims

1. A device for unclogging or fragmenting an embolus comprising:
a catheter (302) having a proximal end, a distal end and a lumen therethrough;
an elongate member (314) at least partially disposed in the lumpen of the catheter, the elongate member having a proximal end and a distal end;
a distal device (304) for unclogging or fragmenting an embolus having a proximal end (312) attached to the elongate member (314); and
a motion control apparatus attached to the proximal end of the elongate member (314), the motion control apparatus being able to impart motion along the axis of the elongate member (314).

2. The device of claim 1, wherein the motion control apparatus is able to impart longitudinal or radial motion or vibration to the distal end of the elongate member (314).

3. The device of claim 1 or 2, wherein the motion control apparatus is able to impart motion to the distal device (304).

4. The device of any of claims 1 to 3, wherein the motion control apparatus is able to impart motion to the distal device (304) at between 1 Hz and 20 kHz.

5. The device of claim 4, wherein the motion control apparatus is able to impart motion to the distal device (304) at between 1 Hz and 150 Hz.

6. The device of any of claims 1 to 5, wherein the range of motion is between 17 mm and 120 mm.

7. The device of any of claims 1 to 6, wherein the catheter is fluidly connected to a vacuum source.

8. A device for unclogging or fragmenting an embolus comprising:
a catheter (206) having a proximal end, a distal end and a lumen therethrough;
an elongate shaft (208) at least partially disposed in the lumen of the catheter, the elongate shaft (208) having a proximal end, a distal end and a lumen therethrough; and
an expandable tip (210) disposed on the distal end of the elongate shaft (208), the expandable tip (210) being adapted to be constrained to fit within the catheter (206); wherein the expandable tip (210), when unconstrained, has an expanded profile and an expanded distal lumen.

9. The device of claim 8, further comprising a vacuum source fluidly connected to the distal end of the catheter (206.

10. The device of claim 8, further comprising a vacuum source fluidly connected to the distal end of the elongate shaft (208).

11. The device of any of claims 8 to 10, further comprising a clot pulling device (212) disposed within the lumen of the elongate shaft (208).

12. The device of claim 11, further comprising a microcatheter (218) having a lumen at least partially disposable in the lumen of the elongate shaft (208), the clot pulling device (212) at least partially disposable in the lumen of the microcatheter.

13. The device of any of claims 8 to 12, wherein the elongate shaft (208) comprises a shape memory polyurethane.

14. The device of any of claims 8 to 12, wherein the elongate shaft (208) comprises a nitinol coiled sheet catheter.

15. The device of any of claims 8 to 12, wherein the elongate shaft (208) comprises an expandable braid.
